# FASCICULE DE BREVET EUROPEEN

(11) **EP 3 539 955 B1**
(45) Date de publication et mention de la délivrance du brevet: **28.07.2021**
(21) Numéro de dépôt: 19161551.7
(22) Date de dépôt: 08.03.2019
(51) Int. Cl.: C07D 401/04, C07D 403/10, C07D 257/12

(54) **PROCÉDÉ DE PRÉPARATION DE TÉTRAZINES**
VERFAHREN FÜR DIE HERSTELLUNG VON TETRAZINEN
PROCESS FOR THE PREPARATION OF TETRAZINES

(30) Priorité: 12.03.2018 FR 1852111
(43) Date de publication de la demande: 18.09.2019
(73) Titulaire: CENTRE NATIONAL DE LA RECHERCHE SCIENTIFIQUE, 75016 Paris (FR); Ecole Normale Supérieure de Cachan, 94235 Cachan Cedex (FR)
(72) Inventeur: MIOMANDRE, Fabien, 60520 La Chapelle en Serval (FR); CLAVIER, Gilles, 94230 Cachan (FR); AUDEBERT, Pierre, 87000 Limoges (FR); QU, Yangyang, 94230 Cachan (FR)
(74) Mandataire: Lavoix

(56) Documents cités:
- QUINTON ET AL.: "Triphenylamine/tetrazine based p-conjugated systems as molecular donors for organic solar cells", NEW J. CHEM., vol. 39, 2015, pages 9700-9713, XP002784531,
- LIM ET AL: "Synthesis of Symmetric 3,6-Disubstituted-1,2,4,5-Tetrazines using an Activated Catalyst Prepared by the Reaction of Copper Nitrate with Excess Zinc in the Presence of Hydrazine Monohydrate", BULL. KOREAN CHEM. SOC., vol. 16, no. 4, 1995, pages 374-377, XP055394027,
- M. O. ABDEL-RAHMAN ET AL.: "A direct synthesis of dihydrotetrazines", TETRAHEDRON LETTERS, vol. 35, 1968, pages 3871-3872, XP002784532,
- CSERÉP ET AL.: "Synthesis and evaluation of nicotinic acid derived tetrazines for bioorthgonal labeling", SYNTHESIS, vol. 47, 27 mai 2015 (2015-05-27), pages 2738-2744, XP002784533,

## Description

La présente invention a pour objet un nouveau procédé de préparation de tétrazines.

A ce jour, les tétrazines sont préparées par un procédé en deux étapes comprenant l'addition d'hydrazine à des précurseurs nitriles, suivie de l'oxydation de la 1,2-dihydrotétrazine résultante (a) K. A. Hofmann, O. Ehrhart, Ber. Dtsch. Chem. Ges. 1912, 45, 2731-2740; b) T. Curtius, A. Hess, J. Prakt. Chem. 1930, 125, 40-53). Ces procédés permettent de transformer la plupart des nitriles aromatiques en tétrazines 3,6-disubstituées symétriques correspondantes, mais ne marchent pas avec les nitriles aliphatiques (a) N. Saracoglu, Tetrahedron 2007, 63, 4199-4236; b) M. O. Abdel-Rahman, M. A. Kira, M. N. Tolba, Tetrahedron Lett. 1968, 9, 3871-3872). En outre, il est difficile de préparer des tétrazines 3,6-disubstituées asymétriques selon cette approche classique, en raison de la formation de mélanges de produits symétriques et asymétriques, et d'une quantité accrue de sous-produits, ce qui entraîne des procédures compliquées de purification et des rendements faibles, typiquement de l'ordre de 10%-20% (M. R. Karver, R. Weissleder, S. A. Hilderbrand, Bioconjugate Chem. 2011, 22, 2263-2270).

Plus récemment, une procédure one-pot avec catalyse métallique a été mise au point pour préparer des tétrazines asymétriques avec des rendements élevés (J. Yang, M. R. Karver, W. Li, S. Sahu, N. K. Devaraj, Angew. Chem. Int. Ed. 2012, 51, 5222-5225). Cependant, ce procédé présente de nombreux inconvénients, notamment en raison de l'utilisation d'hydrazine pure, qui est un réactif gazeux hautement toxique, cancérigène et très délicat à manipuler, ainsi que d'un catalyseur métallique coûteux. Il est fait aussi référence à Quinton , "New J. Chem.", 2015, 39, pages 9700-9713.

Il existe donc un besoin d'un procédé de préparation de tétrazines asymétriques, simple et efficace, avec un rendement satisfaisant, et économiquement intéressant.

La présente invention a donc pour but de fournir un procédé de préparation de tétrazines asymétriques, avec des rendements satisfaisants, et avec un coût réduit.

La présente invention a donc pour but de fournir un procédé de préparation de tétrazines asymétriques, ne nécessitant pas l'utilisation de catalyseur.

La présente invention a donc pour but de fournir un procédé permettant d'obtenir des tétrazines asymétriques, particulièrement adaptées au greffage sur des structures biologiques (cellules, virus, protéines...) ou inorganiques (nanoparticules fonctionnelles).

Ainsi, la présente invention concerne un procédé de préparation d'un composé de formule (I) suivante : 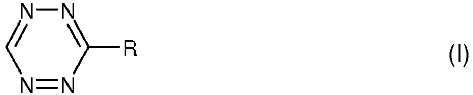 dans laquelle R représente :
- un groupe aryle comprenant de 6 à 10 atomes de carbone, éventuellement substitué,
- un groupe hétéroaryle comprenant de 5 à 10 atomes, dont au moins un hétéroatome choisi parmi O, S ou N, éventuellement substitué, ou
- un groupe alkyle comprenant de 1 à 6 atomes de carbone, substitué par un groupe aryle comprenant de 6 à 10 atomes de carbone,
ledit procédé comprenant :
a) une étape de mélange d'un nitrile de formule (II) suivante :

   R-CN (II)

   R étant tel que défini ci-dessus,
   avec du dichlorométhane et du soufre, dans un alcool,
b) une étape d'addition de monohydrate d'hydrazine au mélange obtenu à l'issue de l'étape a), et mélange de l'ensemble ainsi obtenu,
c) une étape d'oxydation du mélange obtenu à l'issue de l'étape b), et
d) une éventuelle étape d'isolement du composé de formule (I).

Le procédé de l'invention permet donc d'obtenir des tétrazines portant un hydrogène sur l'un des deux carbones tétraziniques, ainsi que, sur l'autre carbone accessible de la molécule, notamment un groupement phényle, portant des fonctions réactives en chimie organique (alcool, acide carboxylique, amine, etc...) adaptées au greffage sur des structures biologiques (cellules, virus, protéines...) ou inorganiques (nanoparticules fonctionnelles).

Les tétrazines ainsi obtenues sont très intéressantes pour le "cliquage" d'objets d'intérêt biologique, par réaction quantitative, à basse température et dans des fluides biologiques, avec des protéines marquées par des alcènes réactifs (par exemple le transcycloctène).

Le procédé selon l'invention est effectué à partir d'hydrate d'hydrazine, aisé à manipuler, et sans catalyseur.

Ce nouveau procédé constitue donc une amélioration importante du procédé existant, et conduit à une baisse très importante du prix de revient des tétrazines ainsi préparées dans un objectif commercial, par le fait de limiter le temps de travail, le coût des réactifs, et, surtout, les exigences en matière de sécurité qui reviennent extrêmement cher.

Les tétrazines obtenues selon le procédé de l'invention répondent à la formule (I) susmentionnée.

Dans la formule (I), R est un groupe aryle, hétéroaryle ou arylalkyle comme indiqué ci-dessus.

Selon l'invention, les groupes aryles sont des groupes aromatiques cycliques comprenant entre 6 et 10 atomes de carbone. A titre d'exemples de groupes aryles, on peut citer les groupes phényle ou naphtyle.

Selon l'invention, le terme "hétéroaryle" désigne un groupe monocyclique ou bicyclique aromatique de 5 à 10 chaînons contenant de 1 à 4 hétéroatomes choisis parmi O, S ou N.

A titre d'exemples, on peut citer les groupes imidazolyle, thiazolyle, oxazolyle, furanyle, thiophényle, pyrazolyle, oxadiazolyle, tétrazolyle, pyridinyle, pyrazinyle, pyrimidinyle, pyridazinyle, indolyle, benzofuranyle, benzothiophényle, benzoxazolyle, benzimidazolyle, indazolyle, benzothiazolyle, isobenzothiazolyle, benzotriazolyle, quinoléinyle, isoquinoléinyle.

A titre d'hétéroaryle comprenant 5 à 6 atomes, dont 1 à 4 atomes d'azote, on peut notamment citer les groupes représentatifs suivants : pyrrolyle, pyrazolyle, 1,2,3-triazolyle, 1,2,4-triazolyle, tétrazolyle et 1,2,3-triazinyle.

On peut également citer, à titre d'hétéroaryle, le thiophényle, l'oxazolyle, le furazanyle, le 1,2,4-thiadiazolyle, le naphthyridinyle, le quinoxalinyle, le phtalazinyle, l'imidazo[1,2-a]pyridine, l'imidazo[2,1-b]thiazolyle, le cinnolinyle, le benzofurazanyle, l'azaindolyle, le benzimidazolyle, le benzothiophényle, le thiénopyridyle, le thiénopyrimidinyle, le pyrrolopyridyle, l'imidazopyridyle, le benzoazaindole, le 1,2,4-triazinyle, l'indolizinyle, l'isoxazolyle, l'isoquinolinyle, l'isothiazolyle, le purinyle, le quinazolinyle, le quinolinyle, l'isoquinolyle, le 1,3,4-thiadiazolyle, le thiazolyle, l'isothiazolyle, le carbazolyle, ainsi que les groupes correspondants issus de leur fusion ou de la fusion avec le noyau phényle.

Selon l'invention, le terme "alkyle" désigne des groupes aliphatiques hydrocarbonés, saturés, linéaires ou ramifiés comprenant, sauf mention contraire, de 1 à 6 atomes de carbone. A titre d'exemples, on peut citer les groupes méthyle, éthyle, n-propyle, isopropyle, butyle, isobutyle, tertbutyle ou pentyle.

Lorsqu'un radical alkyle est substitué par un groupe aryle, on parle de radical "arylalkyle" ou "aralkyle". Les radicaux "arylalkyles" ou "aralkyles" sont des radicaux aryl-alkyl-, les groupes aryles et alkyles étant tels que définis ci-dessus. Parmi les radicaux arylalkyles, on peut notamment citer le radical benzyle ou phénéthyle.

Les radicaux "aryle" et "hétéroaryle" susmentionnés peuvent être substitués par un ou plusieurs substituants. Parmi ces substituants, on peut citer les groupes suivants : amino, hydroxy, thiol, oxo, halogène, alkyle, éventuellement substitué, alcoxy, alkylthio, alkylamino, aryle, aryloxy, arylalcoxy, cyano, trifluorométhyle, carboxy ou carboxyalkyle.

Dans le cadre de la présente invention, le terme "atome d'halogène" désigne les atomes fluor, chlore, brome ou iode.

Selon un mode de réalisation, dans la formule (I), R représente un groupe hétéroaryle tel que défini ci-dessus, éventuellement substitué. De préférence, R est un groupe hétéroaryle non substitué, notamment un groupe pyridinyle.

Selon un mode de réalisation, dans la formule (I), R représente un groupe aryle tel que défini ci-dessus, substitué ou non. De préférence, R est un groupe aryle substitué, notamment un groupe phényle substitué. En particulier, parmi les substituants dudit groupe aryle, de préférence dudit groupe phényle, on peut citer les atomes d'halogène, notamment Br ou Cl, les groupes alkyles, notamment méthyle, les groupes (hétéro)aryles tels que définis ci-dessus, CN, les groupes de formule COORₐ, Rₐ représentant H ou un groupe alkyle comprenant de 1 à 6 atomes de carbone, ou encore les groupes alkyles, notamment méthyles, substitués par OH, COOH ou NR'ₐR'_{b}, R'ₐ et R'_{b}, indépendamment les uns des autres, représentant H ou un groupe alkyle comprenant de 1 à 6 atomes de carbone, ou un groupe protecteur des fonctions amines, par exemple Boc.

Selon un mode de réalisation, dans la formule (I), R représente un groupe -CH2-Ar, Ar représentant un groupe aryle tel que défini ci-dessus et de préférence, un groupe phényle. R peut donc représenter par exemple un groupe benzyle.

Le procédé de l'invention consiste dans un premier temps à mélanger un nitrile de formule (II) telle que définie ci-dessus avec du dichlorométhane et du soufre, dans un alcool.

Selon un mode de réalisation, l'alcool de l'étape a) est un alcool de formule R'OH, R' représentant un groupe alkyle comprenant de 1 à 6 atomes de carbone.

Parmi les alcools, on peut citer par exemple l'éthanol, le butanol ou le propanol, et préférentiellement l'éthanol.

Selon un mode de réalisation, le mélange de l'étape a) est effectué dans un tube pour réacteur à microondes.

Selon un mode de réalisation, l'étape a) est effectuée sous irradiation microondes.

De préférence, dans l'étape a), la teneur en soufre est comprise entre 1 et 4 équivalents, et de préférence entre 1,5 et 2,5 équivalents, par rapport au nitrile de formule (II).

Selon l'étape b), on ajoute du monohydrate d'hydrazine, NH₂NH₂.H₂O, au mélange obtenu à l'issue de l'étape a). Ensuite, l'ensemble est agité.

Selon un mode de réalisation, le mélange obtenu à l'issue de l'étape b) est introduit dans un réacteur à microondes.

Selon un mode de réalisation, le tube dans lequel est effectué l'étape a), et donc également ensuite, l'étape b), est scellé puis introduit dans un réacteur à microondes

Selon un mode de réalisation, le procédé de l'invention comprend, après l'étape b) et avant l'étape c), une étape de chauffage du mélange obtenu à l'issue de l'étape b).

Selon un mode de réalisation, le mélange obtenu à l'issue de l'étape b) est soumis à une étape de chauffage.

De préférence, ladite étape de chauffage est effectuée à une température comprise entre 30°C et 70°C, et de préférence entre 45°C et 55°C.

De préférence, cette étape de chauffage est effectuée pendant une durée comprise entre 3 heures et 48 heures, et de préférence entre 12 heures et 30 heures.

Le mélange obtenu à l'issue de l'étape b), de préférence chauffé, est ensuite soumis à une oxydation. Cette étape consiste notamment à mettre en présence ledit mélange avec un agent oxydant.

Parmi les agents oxydants, on peut citer par exemple tous les nitrites, tels que le nitrite de sodium ou le nitrite d'isoamyle, les oxydes d'azote, les cations métalliques oxydants, tels que, à titre non-limitatif, le cuivre II, le cérium IV, le fer III, le dioxygène, le peroxyde d'hydrogène, les hypohalogènites, les halogènates...

Selon un mode de réalisation, cette étape d'oxydation est effectuée en présence d'oxygène ou de nitrite de sodium. De préférence, l'étape c) consiste à ajouter du nitrite de sodium au mélange obtenu à l'issue de l'étape b), notamment avec de l'eau. De préférence, ensuite, de l'acide acétique est progressivement ajouté au mélange réactionnel.

Selon un mode de réalisation, le procédé de l'invention comprend une étape d'isolement des tétrazines obtenues.

De préférence, l'étape d) consiste en une étape d'extraction, de préférence avec du dichlorométhane, du mélange obtenu à l'issue de l'étape c), éventuellement suivie d'étapes de séchage, évaporation et/ou purification.

Selon un mode de réalisation, le mélange obtenu à l'issue de l'étape c) est extrait par exemple avec du dichlorométhane, puis séché sur du MgSO₄.

Selon un mode de réalisation, le procédé de l'invention comprend en outre, dans le cadre de l'étape d), une étape d'évaporation de tous les solvants, notamment du dichlorométhane, et également de préférence, une étape de purification des tétrazines obtenues sur une colonne de chromatographie.

La présente invention concerne également un composé de formule suivante :

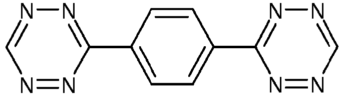

### EXEMPLES

### Mode opératoire général pour la synthèse des tétrazines T1 à T11

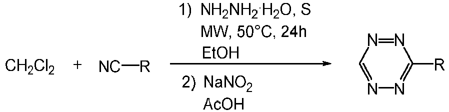

Du benzonitrile (1 mmol, Aldrich Chemicals), du soufre (2 mmoles, 64 mg) et du dichlorométhane (1 mmoles, 63,70 µl) sont mélangés dans 1 ml d'éthanol dans un tube de réaction pour réacteur à microondes. On ajoute alors du monohydrate d'hydrazine pur (8 mmoles, 0,4 ml, Acros Organics), et le mélange est agité. On scelle alors le tube, qui est ensuite introduit dans le réacteur et chauffé à 50°C pendant 24 h. Le mélange réactionnel est ensuite repris avec 3 ml de CH2CI2 puis 10 mmoles (0,69 g) de nitrite de sodium dans 10 ml d'eau sont ajoutées au mélange, et ensuite 20 mmoles (1,14 ml) d'acide acétique sont progressivement ajoutées. Pendant cette opération, le mélange prend la couleur rouge brillante caractéristique des tétrazines. Le mélange est alors extrait avec du dichlorométhane, puis séché sur MgSO₄. Les solvants sont ensuite évaporés et la tétrazine purifiée sur une colonne de chromatographie (LPLC, 4g de silice).

Ce procédé a été appliqué pour l'obtention des différentes tétrazines ci-dessous :

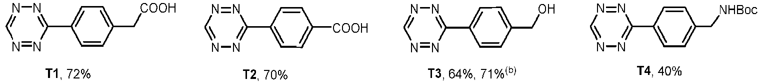

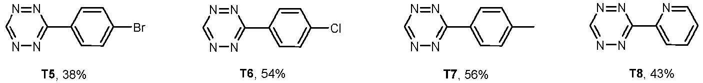

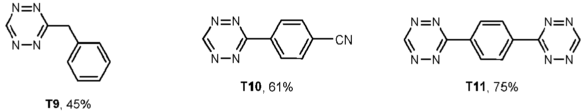

### Caractérisations des produits

### T1

Après chromatographie, un solide rouge brillant est obtenu (CH₂Cl₂ : acide acétique 50:1) (155 mg, rdt: 72%). ¹H NMR (400 MHz, CDCl₃, δ): 10.23 (s, 1H), 8.62 (d, *J* = 8.24 Hz, 2H), 7.55 (d, *J* = 8.24 Hz, 2H), 3.81 (s, 2H); ¹³C NMR (100 MHz, CDCl₃, δ): 175.09, 166.38, 157.99, 138.79, 130.89, 130.65, 128.76, 40.81 ; HRMS [M+H]⁺ m/z calc. pour [C₁₀H₉N₄O₂]⁺ 217.0726, trouvé 217.0722.

### T2

Après chromatographie, un solide rouge est obtenu (éther de pétrole:EtOAc = 1:1) (141 mg, rdt: 70%). ¹H NMR (400 MHz, DMSO-d₆, δ): 10.66 (s, 1H), 8.62 (d, *J* = 8.24 Hz, 2H), 8.21 (d, *J* = 8.24 Hz, 2H); ¹³C NMR (100 MHz, DMSO-d₆, δ): 166.73, 165.10, 158.29, 135.72, 134.37, 130.25, 128.01; HRMS [M-H]⁻ m/z calc. pour [C₉H₅N₄O₂]⁻ 201.0413, trouvé 201.0412.

### T3

Après chromatographie, un solide rouge brillant est obtenu (éther de pétrole : EtOAc = 3:1) (120 mg, rdt: 64%; 133mg, (rdt: 71% si on utilise le chlorhydrate de 4-(aminomethyl)- benzonitrile comme précurseur du nitrile). ¹H NMR (400 MHz, CDCl₃, δ): 10.22 (s, 1H), 8.63 (d, *J* = 8.24 Hz, 2H), 7.62 (d, *J* = 8.24 Hz, 2H), 4.86 (s, 2H); ¹³C NMR (100 MHz, CDCl₃, δ): 166.42, 157.88, 146.37, 130.84, 128.62, 127.54, 64.78; HRMS [M+H]⁺ m/z calc. pour [C₉H₉N₄O]⁺ 189.0776, trouvé 189.0773.

### T4

Après chromatographie, un solide rose est obtenu (CH₂Cl₂ : EtOAc = 100:1) (114 mg, rdt: 40%). ¹H NMR (400 MHz, CDCl₃, δ): 10.22 (s, 1H), 8.60 (d, *J* = 8.24 Hz, 2H), 7.53 (d, *J* = 8.24 Hz, 2H), 4.99 (s, br, 1H), 4.46 (d, *J* = 5.95 Hz, 2H), 1.49 (s, 9H); ¹³C NMR (100 MHz, CDCl₃, δ): 166.43, 157.94, 156.09, 144.79, 130.67, 128.73, 128.28, 80.07, 44.49, 28.53;

### T5

Après chromatographie, un solide rouge est obtenu (éther de pétrole : CH₂Cl₂ = 2:1) (90 mg, rdt: 38%). ¹H NMR (400 MHz, CDCl₃, δ): 10.25 (s, 1H), 8.51 (d, *J* = 8.70 Hz, 2H), 7.76 (d, *J* = 8.70 Hz, 2H); ¹³C NMR (100 MHz, CDCl₃, δ): 166.13, 158.04, 132.90, 130.62, 129.82, 128.65; HRMS [M]⁺ m/z calc. pour [C₈H₅BrN₄]⁺ 235.9704, trouvé 235.9692.

### T6

Après chromatographie, un solide rouge est obtenu (éther de pétrole : CH₂Cl₂ = 2:1) (103 mg, yield: 54%). ¹H NMR (400 MHz, CDCl₃, δ): 10.24 (s, 1H), 8.56 (d, *J* = 8.70 Hz, 2H), 7.59 (d, *J* = 8.70 Hz, 2H); ¹³C NMR (100 MHz, CDCl₃, δ): 165.92, 157.94, 139.92, 130.11, 129.85, 129.63; HRMS [M+H]⁺ m/z calc. pour [C₈H₇ClN₄]⁺ 194.0358, trouvé 194.0354.

### T7

Après chromatographie, un solide rouge est obtenu (éther de pétrole : CH₂Cl₂ = 2:1) (96 mg, rdt: 56%). ¹H NMR (400 MHz, CDCl₃, δ): 10.19 (s, 1H), 8.52 (d, *J* = 8.24 Hz, 2H), 7.42 (d, *J* = 8.24 Hz, 2H), 2.49 (s, 3H); ¹³C NMR (100 MHz, CDCl₃, δ): 166.67, 157.79, 144.15, 130.30, 128.96, 128.41, 21.89; HRMS [M+H]⁺ m/z calc. pour [C₉H₉N₄]⁺ 173.0819, trouvé 173.0822.

### T8

Après chromatographie, un solide rouge est obtenu (éther de pétrole : EtOAc = 1:1) (68 mg, rdt: 43%). ¹H NMR (400 MHz, CDCl₃, δ): 10.37 (s, 1H), 8.99 (d, *J* = 4.58 Hz, 1H), 8.71 (d, *J* = 7.79 Hz, 1H), 8.02 (t, *J* = 7.79 Hz, 1H), 8.71 (dd, *J* = 7.79 Hz, *J* = 4.58 Hz, 1H); ¹³C NMR (100 MHz, CDCl₃, δ): 165.90, 158.50, 151.18, 150.03, 137.66, 126.90, 124.45;

### T9

Après chromatographie, un solide rouge est obtenu (éther de pétrole : CH₂Cl₂ = 2:1) (77 mg, rdt: 45%). ¹H NMR (400 MHz, CDCl₃, δ): 10.21 (s, 1H), 7.43 (d, *J* = 8.24 Hz, 2H), 7.34 (dd, *J* = 8.24 Hz, *J* = 7.33 Hz, 2H), 7.28 (d, *J* = 7.33 Hz, 1H), 4.67 (s, 2H); ¹³C NMR (100 MHz, CDCl₃, δ): 172.07, 158.18, 135.64, 129.40, 129.13, 127.67, 41.93; HRMS [M]⁺ m/z calc. pour [C₁₀H₈N₄]⁺ 172.0750, trouvé 172.0743.

### T10

Après chromatographie, un solide rouge cristallin est obtenu (éther de pétrole : CH₂Cl₂ = 1:1) (111 mg, rdt: 61%). ¹H NMR (400 MHz, CDCl₃, δ): 10.33 (s, 1H), 8.79 (d, *J* = 8.70 Hz, 2H), 7.93 (d, *J* = 8.70 Hz, 2H); ¹³C NMR (100 MHz, CDCl₃, δ): 165.47, 158.28, 135.65, 133.20, 128.82, 118.11, 116.71.;

### T11

Après chromatographie, un solide rouge cristallin est obtenu (éther de pétrole : CH₂Cl₂ = 1:3) (178 mg, rdt: 75%). ¹H NMR (400 MHz, DMSO-d₆, δ): 10.65 (s, 2H), 8.79 (s, 4H); ¹³C NMR (100 MHz, DMSO-d₆, δ): 165.63, 165.10, 158.64, 136.14, 129.19.

## Revendications

1. Procédé de préparation d'un composé de formule (I) suivante : 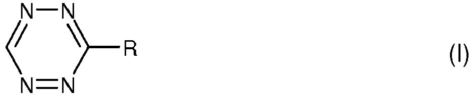 dans laquelle R représente :
- un groupe aryle comprenant de 6 à 10 atomes de carbone, éventuellement substitué,
- un groupe hétéroaryle comprenant de 5 à 10 atomes, dont au moins un hétéroatome choisi parmi O, S ou N, éventuellement substitué, ou
- un groupe alkyle comprenant de 1 à 6 atomes de carbone, substitué par un groupe aryle comprenant de 6 à 10 atomes de carbone,
ledit procédé comprenant :
a) une étape de mélange d'un nitrile de formule (II) suivante :
R-CN (II)
R étant tel que défini ci-dessus,
avec du dichlorométhane et du soufre, dans un alcool,
b) une étape d'addition de monohydrate d'hydrazine au mélange obtenu à l'issue de l'étape a), et mélange de l'ensemble ainsi obtenu,
c) une étape d'oxydation du mélange obtenu à l'issue de l'étape b), et
d) une éventuelle étape d'isolement du composé de formule (I).

2. Procédé selon la revendication 1, dans lequel l'alcool de l'étape a) est un alcool de formule R'OH, R' représentant un groupe alkyle comprenant de 1 à 6 atomes de carbone.

3. Procédé selon la revendication 1 ou 2, dans lequel l'étape c) d'oxydation est effectuée en présence d'oxygène ou de nitrite de sodium.

4. Procédé selon l'une quelconque des revendications 1 à 3, dans lequel l'étape a) est effectuée sous irradiation microondes.

5. Procédé selon l'une quelconque des revendications 1 à 4, dans lequel, dans l'étape a), la teneur en soufre est comprise entre 1 et 4 équivalents, par rapport au nitrile de formule (II).

6. Procédé selon l'une quelconque des revendications 1 à 5, comprenant, après l'étape b) et avant l'étape c), une étape de chauffage du mélange obtenu à l'issue de l'étape b).

7. Procédé selon la revendication 6, dans lequel l'étape de chauffage est effectuée à une température comprise entre 30°C et 70°C.

8. Procédé selon la revendication 6 ou 7, dans lequel l'étape de chauffage est effectuée pendant une durée comprise entre 3 heures et 48 heures.

9. Procédé selon l'une quelconque des revendications 1 à 8, dans lequel l'étape d) consiste en une étape d'extraction, de préférence avec du dichlorométhane, du mélange obtenu à l'issue de l'étape c), éventuellement suivie d'étapes de séchage, évaporation et/ou purification.

10. Composé de formule suivante :

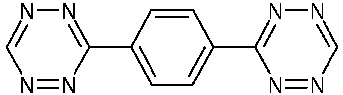

## Patentansprüche

1. Verfahren zum Herstellen einer Verbindung der folgenden Formel (I): 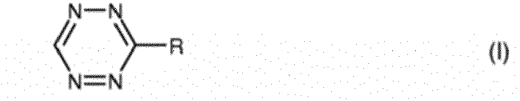 wobei R darstellt:
- eine Arylgruppe, aufweisend 6 bis 10 Kohlenstoffatome, welche eventuell substituiert ist,
- eine Heteroarylgruppe, aufweisend 5 bis 10 Atome, wovon wenigstens ein Heteroatom ausgewählt ist aus O, S oder N, welche eventuell substituiert ist, oder
- eine Alkylgruppe, aufweisend 1 bis 6 Kohlenstoffatome, welche substituiert ist durch eine Arylgruppe, aufweisend 6 bis 10 Kohlenstoffatome,
wobei besagtes Verfahren aufweist:
a) einen Schritt des Mischens eines Nitrils der folgenden Formel (II):
R-CN (II)
wobei R wie oben definiert ist,
mit Dichlormethan und Schwefel, in einem Alkohol,
b) einen Schritt des Zufügens von Hydrazin-Monohydrat zur am Ende des Schritts a) erlangten Mischung und Mischens der so erlangten Menge,
c) einen Schritt des Oxidierens der am Ende des Schritts b) erlangten Mischung und
d) einen eventuellen Schritt des Isolierens der Verbindung der Formel (I).

2. Verfahren gemäß Anspruch 1, wobei der Alkohol des Schritts a) ein Alkohol der Formel R'OH ist, wobei R' eine Alkylgruppe mit 1 bis 6 Kohlenstoffatomen darstellt.

3. Verfahren gemäß Anspruch 1 oder 2, wobei der Schritt c) des Oxidierens in Anwesenheit von Sauerstoff oder Natriumnitrit durchgeführt wird.

4. Verfahren gemäß irgendeinem der Ansprüche 1 bis 3, wobei der Schritt a) unter Mikrowellenstrahlung durchgeführt wird.

5. Verfahren gemäß irgendeinem der Ansprüche 1 bis 4, wobei im Schritt a) der Schwefelanteil zwischen 1 und 4 Äquivalenten liegt mit Bezug auf das Nitril der Formel (II).

6. Verfahren gemäß irgendeinem der Ansprüche 1 bis 5, aufweisend, nach dem Schritt b) und vor dem Schritt c), einen Schritt des Erwärmens der am Ende des Schritts b) erlangten Mischung.

7. Verfahren gemäß Anspruch 6, wobei der Schritt des Erwärmens bei einer Temperatur zwischen 30°C und 70°C durchgeführt wird.

8. Verfahren gemäß Anspruch 6 oder 7, wobei der Schritt des Erwärmens während einer Dauer zwischen 3 Stunden und 48 Stunden durchgeführt wird.

9. Verfahren gemäß irgendeinem der Ansprüche 1 bis 8, wobei der Schritt d) aus einem Schritt des Extrahierens, vorzugsweise mit Dichlormethan, der am Ende des Schritts c) erlangten Mischung besteht, eventuell gefolgt von Schritten des Trocknens, Eindampfens und/oder Aufreinigens.

10. Verbindung der folgenden Formel:

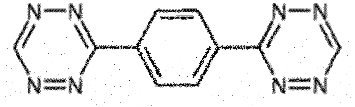

## Claims

1. Method of preparing a compound of the following Formula (1): 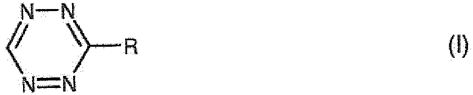 in which R represents:
- an aryl group comprising from 6 to 10 carbon atoms, possibly substituted,
- a heteroaryl group comprising from 5 to 10 atoms, including at least one heteroatom chosen from O, S or N, possibly substituted, or
- an alkyl group comprising from 1 to 6 carbon atoms, substituted by an aryl group comprising from 6 to 10 carbon atoms,
said method comprising:
a) a step of mixing a nitrile of the following Formula (II):
R-CN (II)
R being as defined above,
with dichloromethane and sulphur, in an alcohol,
b) a step of adding hydrazine monohydrate to the mixture obtained at the end of step a), and mixing the assembly thus obtained,
c) a step of oxidising the mixture obtained at the end of step b), and
d) a possible step of isolating the compound of Formula (I).

2. Method according to claim 1, in which the alcohol of step a) is an alcohol of formula R'OH, R' representing an alkyl group comprising from 1 to 6 carbon atoms.

3. Method according to claim 1 or 2, in which step c) of oxidising is effected in the presence of oxygen or sodium nitrite.

4. Method according to any of claims 1 to 3, in which step a) is effected under microwave irradiation.

5. Method according to any of claims 1 to 4, in which, in step a), the content of sulphur is between 1 and 4 equivalents, relative to the nitrile of formula (II).

6. Method according to any of claims 1 to 5, comprising, after step b) and before step c), a step of heating the mixture obtained at the end of step b).

7. Method according to claim 6, in which the step of heating is effected at a temperature between 30°C and 70°C.

8. Method according to claim 6 or 7, in which the step of heating is effected over a duration between 3 hours and 48 hours.

9. Method according to any of claims 1 to 8, in which step d) consists of a step of extracting, preferably with dichloromethane, the mixture obtained at the end of step c), possibly followed by steps of drying, evaporation and/or purification.

10. Compound of the following formula:
